# EUROPÄISCHE PATENTANMELDUNG

(11) **EP 1 239 030 A1**
(43) Veröffentlichungstag der Anmeldung: **11.09.2002**
(21) Anmeldenummer: 02005153.8
(22) Anmeldetag: 07.03.2002
(51) Int. Cl.: C12M 1/107

(54) **Anlage zur Erzeugung von Biogas unter Verwendung von organischen Feststoffen**

(30) Priorität: 07.03.2001 DE 10110861; 21.03.2001 DE 20104931 U
(71) Anmelder: JACOBS, HANS-HERMANN, 29643 NEUENKIRCHEN (DE)
(72) Erfinder: JACOBS, HANS-HERMANN, 29643 NEUENKIRCHEN (DE)
(74) Vertreter: WALTHER, WALTHER & HINZ Patentanwälte

(57) **Zusammenfassung**

Gegenstand der Erfindung ist eine Anlage zur Erzeugung von Biogas unter Verwendung von organischen Feststoffen, mit einem ein Gärmedium (10) aufnehmenden Gärbehälter (12) und mit einem die zur Vergärung vorgesehenen, oprganische Feststoffe aufnehmenden Vorratsbehälter (20). Eine Anlage zur Erzeugung von Biogas unter Verwendung von kostengünstig zu erzeugenden organischen Feststoffen zu schaffen, die eine personalsparende Beschickung des Gärbehälters mit Feststoffen ermöglicht, wird durch eine die Feststoffe selbstätig, kontrolliert und gesteuert in den Gärbehälter (12) einbringende Beschickungsvorrichtung (18) erreicht.

## Beschreibung

Die vorliegende Erfindung betrifft eine Anlage zur Erzeugung von Biogas unter Verwendung von organischen Feststoffen gemäß dem Oberbegriff des Anspruches 1 und/oder 4 und ein Verfahren gemäß dem Oberbegriff des Anspruches 13, 14 und/oder 15.

Bei der Erzeugung von Biogas (überwiegend Methan) werden biologische Abfälle wie beispielsweise Gülle, Altfette, Bioabfälle, Essensreste oder dergleichen in einem Gärbehälter vergoren. Da diese Rohstoffe nur begrenzt zur Verfügung stehen, werden zunehmend schüttbare, organische Feststoffe, wie beispielsweise Mais, Rüben, Obst, Gemüse, Getreide oder andere Agrarprodukte eingesetzt. Diese schüttbaren Feststoffe können kostengünstig auf stillgelegten und/oder kontaminierten Agrarflächen angebaut werden, um so einen kostengünstigen Rohstoff zu erhalten. Das dabei entstehende Biogas wird anschließend in einem Verbrennungsmotor, vorzugsweise einem auf Schwachgas umgerüsteten Dieselmotor, verbrannt und über einen Generator in elektrische Energie umgewandelt. Die dabei entstehende Abwärme des Motors wird, wenn überhaupt, nur extensiv genutzt.

Aus der DE 200 11 785 U1 ist eine Biogasanlage bekannt, bei der über ein Förderrohr organische Feststoffe aus einem Einwurfschacht in einen Fermenter transportiert werden. Da die im Einwurfschacht befindlichen Feststoffe mit aus dem Fermenter austretenden Gasen oder bereits vorgegärten Feststoffen in Kontakt kommen können, darf im Einwurfschacht nur eine geringe Menge an organischen Feststoffen gelagert werden. Ansonsten wurden unter den gelagerten Feststoffe bereits unerwünschte chemische und/oder biologische Prozeße ausgelöst werden. Folglich muß die Biogasanlage stets von geeignetem Bedienpersonal betreut werden, insbesondere müssen in kurzen Zeitintervallen von einigen Stunden regelmäßig weitere Feststoffe in den Einwurfschacht eingebracht werden.

Davon ausgehend liegt der vorliegenden Erfindung die Aufgabe zugrunde eine Anlage zur Erzeugung von Biogas unter Verwendung von kostengünstig zu erzeugenden organischen Feststoffen zu schaffen, die eine personalsparende Beschickung des Gärbehälters mit diesen Feststoffen ermöglicht.

Als erste technische Lösung dieser Aufgabe wird erfindungsgemäß eine Anlage mit den Merkmalen des Anspruches 1 und ein Verfahren mit den Merkmalen des Anspruches 13 und 15 vorgeschlagen. Vorteilhafte Weiterbildungen der Anlage sind den entsprechenden Unteransprüchen zu entnehmen.

Eine nach dieser technischen Lehre ausgebildete Anlage hat den Vorteil, dass die Beschickungsvorrichtung eine vorher eingestellte Menge der organischen Feststoffe genau kontrolliert und selbsttätig, d. h. ohne Zutun des Bedienpersonals in den Gärbehälter einbringt. Folglich muss das Bedienpersonal lediglich dafür Sorge tragen, dass im Vorratsbehälter genügend Feststoffe zur Verfügung stehen. Dabei ist es möglich, den Vorratsbehälter so zu dimensionieren, dass ein für mehrere Tage ausreichender Vorrat eingefüllt werden kann. Hierdurch können einerseits entsprechende Maschinen zum effektiven Transport der Feststoffe eingesetzt werden und andererseits kann beispielsweise am Wochenende auf Bedienpersonal verzichtet werden.

Weitere Vorteile bestehen darin, dass durch die Anordnung eines Schubbodens im Vorratsbehälter eine vorbestimmte Menge der Feststoffe zuverlässig und gleichmäßig ausgetragen werden kann.

Eine zwischen dem Vorratsbehälter und der Preßvorrichtung vorgesehen Schubstange hat den Vorteil, dass hierdurch ein präziserer und weniger Verlust aufweisender Transport der Feststoffe vom Vorratsbehälter in die Preßvorrichtung erreicht wird, da die Schubstange die auftretenden organischen Feststoffe gleich portioniert und kontrolliert in die Preßvorrichtung transportiert. Durch die vorteilhafterweise vorgeschaltete Reißwelle werden etwaige Klumpen aufgelöst und größere Feststoffe zerkleinert, so dass eine genaue Dosierung möglich ist und so dass die in die Pressvorrichtung eintretenden Feststoffe in einfacher Weise zu einer zusammenhängenden und geschlossenen Masse verdichtet werden können.

Durch die Anbringung eines entsprechenden Schiebers über der Austragsöffnung des Vorratsbehälters kann die ins Freie gelangende Menge der organischen Feststoffe reguliert werden.

Als zweite technische Lösung dieser Aufgabe wird erfindungsgemäß eine Anlage mit den Merkmalen des Anspruches 4 und ein Verfahren mit den Merkmalen des Anspruches 14 vorgeschlagen. Vorteilhafte Weiterbildungen der Anlage sind den Unteransprüchen 5 bis 12 zu entnehmen.

Eine nach dieser technischen Lehre ausgebildete Biogasanlage hat den Vorteil, dass die organischen Feststoffe Mittels der Preßvorrichtung verdichtet und in das Preßrohr eingedrückt werden. Hierdurch bilden die verdichtenden Feststoffe eine Art Pfropfen und verhindern, dass Gase und/oder üble Gerüche aus dem Gärbehälter ins Freie, respektive in den Vorratsbehälter, dringen. Dieser Effekt wird dadurch noch weiter verstärkt, dass das Aufnahmerohr der Preßvorrichtung einen größeren Druchmesser, als das Preßrohr selbst aufweist. Hierdurch muss dieser Pfropfen beim Austritt des Aufnahmerohres und bei dem Eintritt in das Preßrohr nochmals verdichtet werden, so dass der Pfropfen noch weniger Gase und/oder Gerüche durchlässt.

Wenn also keine Gase oder Feststoffe mit den zu bevorrateten Feststoffen in Kontakt kommen, können dort auch keine chemischen oder biologischen Prozesse in Gang gesetzt werden, so dass die Feststoffe länger lagerfähig bleiben. Aus diesem Grunde ist eine Bevorratung der Feststoffe und eine automatisierte und somit personalsparende Beschickung möglich mit der Folge, dass hierdurch Arbeitskräfte und Kosten eingespart werden können.

Durch die Verjüngung des Querschnittes am Übergang von Pressrohr zu Aufnahmerohr wird eine zusätzliche Verdichtung erreicht, wodurch eine weitere und verbesserte Abdichtung des Pressrohres zum Gärbehälter erreicht wird.

Die Einbindung des Preßrohres in den Gärbehälter unterhalb der Schwimmdecke hat den Vorteil, dass durch das Preßrohr keine im Gärbehälter befindlichen Gase austreten können, da die Mündung des Preßrohres vollständig vom Gärmedium abgedeckt wird.

Die Anordnung der Preßvorrichtung höher als das Gärmedium hat den Vorteil, dass kein Gärmedium über das Preßrohr und die Preßvorrichtung aus dem Gärbehälter austreten kann.

Das Erwärmen der Feststoffe außerhalb des Gärbehälters hat den Vorteil, dass vorzeitig ein Teilaufschluß der langkettigen Kohlenstoffverbindungen erfolgt. Hierdurch wird der Gärprozess beschleunigt und es wird eine vollständigere Vergärung ermöglicht, was zu einer um bis zu 15% höheren Biogasausbeute führt. Insbesondere wird hierdurch der CH₄ Anteil des Biogases erhöht, was auch zu einer verbesserten Produktqualität führt. Dabei hat es sich als vorteilhaft herausgestellt, die Feststoffe auf ca. 70° C zu erwärmen.

Werden die derart erwärmten organischen Feststoffe im erwärmten Zustand in den Gärbehälter eingebracht, so braucht dieser entsprechend weniger aufgeheizt zu werden, was zu einer Einsparung von Wärmeenergie führt.

Noch ein Vorteil besteht darin, dass zur Erwärmung der organischen Feststoffe die in der Regel im Überfluß vorhandene Abwärme der Biogas-Verbrennungsmotoren verwendet werden kann, so dass keine zusätzlichen Energiekosten anfallen.

Ein weiterer Vorteil der vorzeitigen Erwärmung der organischen Feststoffe besteht darin, dass hierdurch eine Hygienisierung und/oder eine Pasteurisierung der Feststoffe erfolgt, so dass die in den Gärbehälter eingebrachten Feststoffe hygienisch einwandfrei sind. Hierdurch wird eine Verunreinigung des Gärmediums mit unerwünschten Bakterien vermieden.

Die Anbringung einer Sprüheinrichtung im Preßrohr hat den Vorteil, dass an dieser Stelle ein flüssiges Gleitmittel in das Preßrohr eingebracht werden kann, um den Reibungswiderstand des unter Druck stehenden Pfropfens aus Feststoffen zu reduzieren, so dass die organischen Feststoffe unter Vermeidung einer Verstopfung durch das Preßrohr transportiert werden können. Dabei hat es sich als vorteilhaft erwiesen als Gleitmittel Wasser einzusetzen, unter anderem da die Feststoffe spätestens im Gärbehälter ohnehin mit Wasser versetzt werden müssen.

Die Anbringung im Knick einer Krümmung des Preßrohres hat den Vorteil, dass hierbei das Gleitmittel an einem Ort des größten Widerstandes eingebracht werden kann, was zu einer sehr effektiven Schmierung der verdichteten Feststoffe führt, insbesondere wenn die Sprühvorrichtung am Aussenradius des Preßrohres angebracht ist.

Der chargenweise Betrieb der Anlage hat den Vorteil, dass die Feststoffe einer Charge einen gleichlangen Zeitraum im Pressrohr verweilen, so dass alle diese Feststoffe beim Eintritt in den Gärbehälter die gleiche Temperatur aufweisen und gleichermaßen vorgegärt sind. Mit derart gleich vorbereiteten Feststoffen wird die eingentlich Gärung beschleinigt, was zu einer höheren Gasausbeute der Biogasanlage führt.

Es versteht sich, dass zu den erfindungsgemäß genannten organischen Feststoffen auch zähfließende oder pastöse Materialien zählen.

Weitere Vorteile der erfindungsgemäßen Anlage ergeben sich aus der beigefügten Zeichnung und den nachfolgend beschriebenen Ausführungsformen. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Es zeigen:
- Fig. 1: eine schematische dargestellte Seitenansicht einer ersten erfindungsgemäßen Anlage;
- Fig. 2: eine schematische dargestellte Draufsicht einer zweiten erfindungsgemäßen Anlage;
- Fig. 3: eine teilweise Frontansicht auf die Anlage gemäß Fig. 2;
- Fig. 4: eine geschnitten dargestellte Seitenansicht eines Preßrohres mit Sprühvorrichtung;
- Fig. 5: das Preßrohr mit Sprühvorrichtung gemäß Fig. 4, geschnitten entlang Linie V - V in Fig. 4.

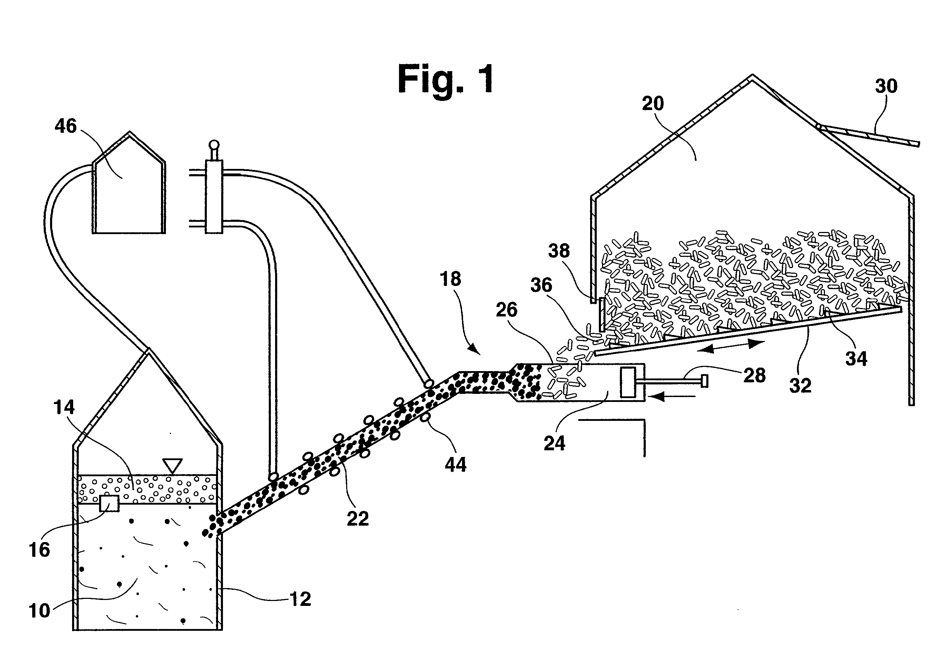

Die in Figur 1 dargestellte erste Ausführungsform einer erfindungsgemäßen Biogasanlage umfasst einen teilweise mit Gärmedium 10 gefüllten Fermenter oder Gärbehälter 12, wobei auf dem Gärmedium 10 eine schwimmende Schwimmdecke 14 ausgebildet ist. Auf dem Gärmedium 10 schwimmt außerdem ein schwimmendes Rührwerk 16, welches die Oberfläche des Gärmediums 10 regelmäßig umrührt.

Weiterhin umfasst diese erste Biogasanlage eine Beschickungsvorrichtung 18 und einen Vorratsbehälter 20, wobei die Beschickungsvorrichtung 18 ein unterhalb der Schwimmdecke 14 in den Gärbehälter 12 mündendes Preßrohr 22 umfasst, an dessen anderem Ende sich ein Aufnahmerohr 24 anschließt. In diesem Aufnahmerohr 24 befindet sich eine Öffnung 26 zur Aufnahme der organischen Feststoffe und ein Preßkolben 28. Durch die Öffnung 26 gelangt eine vorbestimmte Menge der Feststoffe in das Aufnahmerohr 24, in dem diese dann durch den Preßkolben 28 zusammengepreßt werden. Mit der Zeit verdichten sich die Feststoffe derart, dass quasi ein Pfropfen entsteht, so dass aus dem Gärbehälter 12 keine Gase oder Gerüche ins Freie gelangen können. Dieser Pfropfen gelangt vom etwas breiteren Aufnahemrohr 24 in das schmalere Preßrohr 22, so dass sich die organischen Feststoffe hier weiter verdichten und einen noch dichteren Pfropfen bilden. Dabei ist die Preßvorrichtung mit dem Aufnahmerohr 24 und dem Preßkolben 28 höher als das Gärmedium 10 angeordnet, damit das Gärmedium 10 nicht über das Preßrohr 22 aus dem Gärbehälter 12 herausfließen kann. Gleichzeitig ist aber auch die Mündung des Preßrohres 22 unterhalb der Schwimmdecke 14 angeordnet, damit keine etwaig im Gärbehälter 12 vorhandenen Gase durch das Preßrohr 22 ins Freie gelangen können. Hierdurch entsteht ein Sauerstoffabschluss.

Im Vorratsbehälter 20 ist eine größere Menge Mais, Gräser oder andere organische Feststoffe gelagert, so dass die Biogasanlage hiermit mehrere Tage oder gar Wochen betrieben werden kann. Dabei wird der Mais oder die anderen organischen Feststoffe durch eine Klappe 30 in den Vorratsbehälter 20 eingefüllt und dort gelagert. Unter den Feststoffen ist ein sogenannter Schubboden 32 vorgesehen, auf dessen Oberseite eine Vielzahl von im Querschnitt dreieckförmigen Mitnehmern 34 angebracht ist. Dieser Schubboden 32 ragt in eine im Vorratsbehälter 20 vorgesehene Austragsöffnung 36 hinein und kann zur Austragsöffnung 36 hin und von dieser weg bewegt werden. Während diesen Hin- und Herbewegungen gleiten die Mitnehmer 34 mit ihrer flachen Seite unter den Feststoffen hinweg und nehmen eine gewisse Menge dieser Feststoffe mit ihren steilen Seiten während der nächsten Bewegung nach vorne mit. Hierdurch wird eine gewisse Menge der organischen Feststoffe ausgetragen. Dabei kann die auszutragende Menge durch einen am Vorratsbehälter 20 angebrachten, vertikal verfahrbaren Schieber 38 reguliert werden. Die aus der Austragsöffnung 36 heraustretenden organischen Feststoffe fallen dann direkt durch die Öffnung 26 in das Aufnahmerohr 24 und werden dort durch den Preßkolben 28 verdichtet. In einer hier nicht dargestellten Ausführungsform ist auf dem Aufnahmerohr 24 ein Trichter vorgesehen, so dass die Feststoffe zuverlässig und verlustfrei in die Preßvorrrichtung eingeleitet werden.

Um das Preßrohr 22 herum ist ein rohrförmiger Heizmantel 44 vorgesehen, der das Preßrohr 22 zumindest in einem Teilabschnitt umhüllt, um die darin befindlichen Feststoffe auf ca. 70° C zu erwärmen. Hierdurch werden in den Feststoffen die langkettigen Kohlenstoffverbindungen teilweise vorzeitig aufgeschlossen und hygienisiert, was unter anderem zu einer höheren Gasausbeute führt. Dieser Heizmantel 44 wird mit vom Verbrennungsmotor 46 erwärmten Abwasser gespeist, wobei das Abwasser zwischen Heizmantel 44 und Preßrohr 22 fließt. Das System arbeitet in Zeitintervallen, das heißt, der Preßrohrinhalt wird ähnlich einem Chargenbetrieb zweistündlich gefüllt, erhitzt und dann durch die nächste Charge herausgedrückt. Dieses System ist somit gleichzeitig ein Feststoffhygienisierungs- (bzw. -pasteurisierungs-) System.

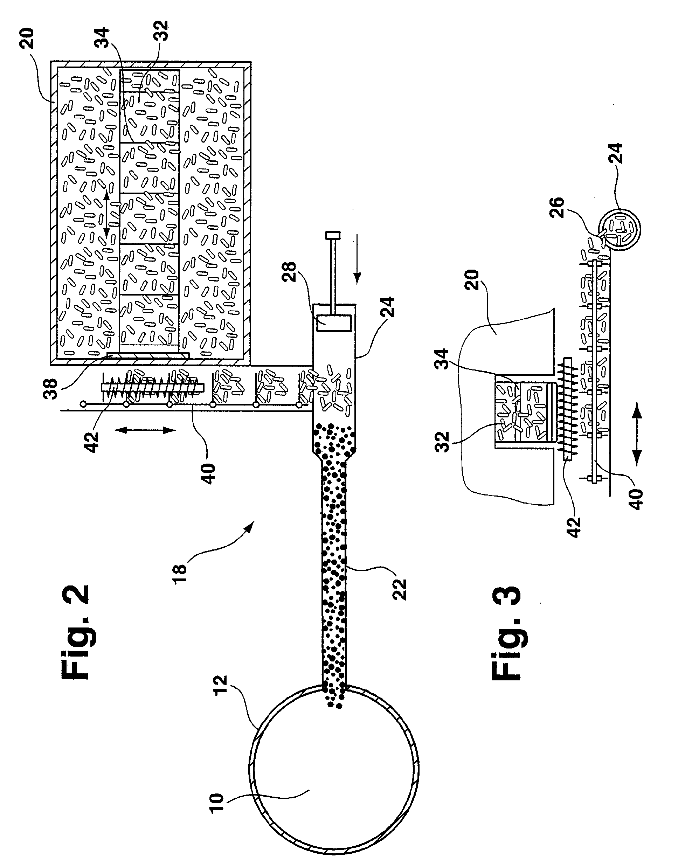

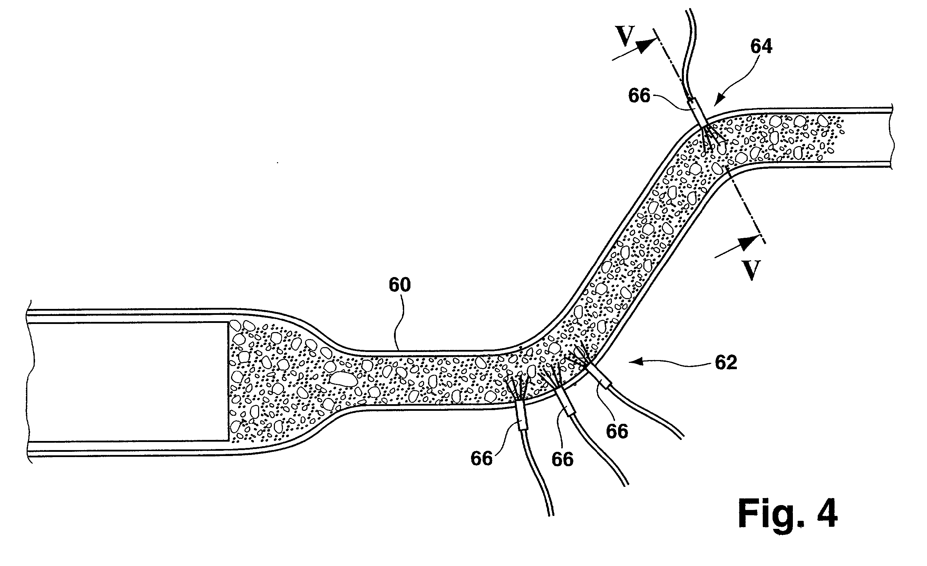

Die in den Figuren 2 und 3 dargestellte zweite Ausführungsform einer erfindungsgemäßen Biogasanlage entspricht in wesentlichen Teilen der in Figur 1 dargestellten Ausführungsform, jedoch ist in dieser zweiten Ausführungsform zwischen dem Vorratsbehälter 20 und dem Aufnahmerohr 24 der Preßvorrichtung eine sogenannte Schubstange 40 zwischengeschaltet und es fehlt der Heizmantel 44. An dieser Schubstange 40 ist eine Vielzahl von in eine Richtung wegdrehbaren Mitnehmern 42 vorgesehen, die die aus der Austragsöffnung 36 herausfallenden Feststoffe in kleineren Portionen aufnehmen und über die Öffnung 26 ins Aufnahmerohr 24 transportieren. Dabei ist oberhalb der Schubstange 40 eine Reißwelle 42 angeordnet, mit der die zum Vorratsbehälter 20 heraustretenden Feststoffe aufgelockert werden, so dass eine die Öffnung 26 des Aufnahmerohres 24 verstopfende Klumpenbildung vermieden wird.

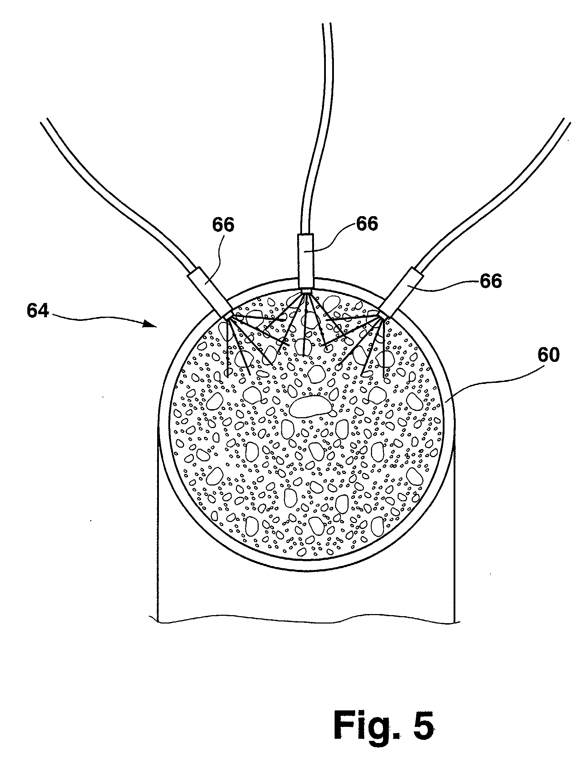

In den Figuren 4 und 5 ist ein alternatives Preßrohr 60 dargestellt, dessen Verlauf zwei 45 ° Krümmungen 62 und 64 aufweist. Im Außenbereich dieser Krümmungen 62, 64 sind je drei Sprühvorrichtungen 66 angebracht, mittels derer Wasser in das Preßrohr 60 eingesprüht werden kann. Dabei sind die Sprühvorrichtungen 66 im Bereich der Krümmung 62 in Vorschubrichtung hintereinander angeordnet, während die Sprühvorrichtungen 66 im Bereich der Krümmung 64 nebeneinander angeordnet sind. Die Sprühvorrichtungen 66 sind in der Krümmung 64 genau im Knick angeordnet, weil dort die größte Gefahr einer Verstopfung herrscht.

Mit der Sprühvorrichtung 66 wird bei Bedarf, beispielsweise bei einer Verstopfung des Preßrohres 60, eine Flüssigkeit, vorzugsweise Wasser, ins Innere des Preßrohres 60 eingesprüht. Diese Flüssigkeit weicht den Pfropfen aus Feststoffen in diesem Bereich partiell auf und/oder dient als Gleitmittel zwischen der Außenwand des Preßrohres und dem Pfropfen aus Feststoffen.

In allen Ausführungsformen der erfindungsgemäßen Biogasanlage erfolgt die Beschickung des Gärbehälters wie folgt:

Zunächst einmal wird eine größere Menge an Mais, Gras oder anderen zur Vergärung vorgesehenen organischen Feststoffen, beispielsweise mittels eines Schaufelladers, Traktors oder Kippladers, in den Vorratsbehälter 20 eingefüllt. Aus diesem Vorratsbehälter 20 wird unter Zuhilfenahme des sich vor- und zurückbewegenden Schubbodens 32 regelmäßig eine kleinere Menge dieser organischen Feststoffe durch die Austragsöffnung 36 aus dem Vorratsbehälter 20 heraus transportiert. Dabei kann die heraus zu transportierende Menge über den vertikalen Schieber 38 eingestellt werden. Die ausgetragenenen Feststoffe gelangen dann über die Schubstange 40 oder direkt in das Aufnahmerohr 24 und werden dort durch den Preßkolben 28 zusammengepreßt und verdichtet. Durch nachfolgende Feststoffe werden die zuerst in das Aufnahmerohr 24 gelangten Feststoffe weiter verdichtet und ins Preßrohr vorgeschoben. Da das Preßrohr 22 einen kleineren Durchmesser als das Aufnahmerohr 24 aufweist, werden die Feststoffe hier weiter verdichtet, so dass der Austritt von Gasen, Gerüchen oder dergleichen aus dem Gärbehälter verhindert wird. Mit der Zeit werden die Feststoffe weiter vorangetrieben und gelangen dann über das Preßrohr 22 in den Gärbehälter 12. Hierbei kann die zu transportierende Menge der Feststoffe so eingestellt werden, dass immer eine zur Gärung ausreichende Menge Feststoffe nachgeliefert wird. In dem Preßrohr 22 werden die organischen Feststoffe auf ca. 70° C erwärmt und gelangen mit dieser Temperatur in den Gärbehälter, in dem die endgültige Vergärung erfolgt.

In einer anderen, hier nicht dargestellten Ausführungsform ist im Vorratsbehälter anstelle des Schubbodens ein Kratzboden vorgesehen, mit dem die Feststoffe geziehlt und dosiert ausgetragen werden.

### Bezugszeichenliste:

- 10: Gärmedium
- 12: Gärbehälter
- 14: Schwimmdecke
- 16: Rührwerk
- 18: Beschickungsvorrichtung
- 20: Vorratsbehälter
- 22: Preßrohr
- 24: Aufnahmerohr
- 26: Öffnung
- 28: Preßkolben
- 30: Klappe
- 32: Schubboden
- 34: Mitnehmer
- 36: Austragsöffnung
- 38: Schieber
- 40: Schubstange
- 42: Reißwelle
- 44: Heizschlange
- 46: Verbrennungsmotor
- 60: Preßrohr
- 62: Krümmung
- 64: Krümmung
- 66: Sprühvorrichtung

## Patentansprüche

1. Anlage zur Erzeugung von Biogas unter Verwendung von organischen Feaststoffen, mit einem ein Gärmedium (10) aufnehmenden Gärbehälter (12), mit einem die zur Vergärung vorgesehenen Feststoffe aufnehmenden Vorratsbehälter (20) und mit einer die Feststoffe selbstätig in den Gärbehälter (12) einbringenden Beschickungsvorrichtung (18)
**dadurch gekennzeichnet,**
**dass** zur Austragung der organischen Fesststoffe im Vorratsbehälter (20) ein Schubboden (32) oder ein Kratzboden vorgesehen ist, wobei der Schubboden (32) oder der Kratzboden die Feststoffe durch eine in einer Wandung des Vorratsbehälters (20) befindliche Austragsöffnung (36) austrägt, und dass die Beschickungsvorrichtung (18) eine Schubstange (40) umfasst, mit der die aus einer Austragsöffnung (36) des Vorratsbehälters (20) austretenden Feststoffe weiter transportiert werden.

2. Anlage nach Anspruch 1,
**dadurch gekennzeichnet,**
**dass** der Schubstange (40) vorgeschaltet eine Reißwelle (42) vorgesehen ist.

3. Anlage nach einem der vorangehenden Ansprüche,
**dadurch gekennzeichnet,**
**dass** an der Austragsöffnung (36) ein vertikal verfahrbarer Schieber (38) angebracht ist.

4. Anlage zur Erzeugung von Biogas unter Verwendung von organischen Feststoffen, mit einem ein Gärmedium (10) aufnehmenden Gärbehälter (12), mit einem die zur Vergärung vorgesehenen Feststoffe aufnehmenden Vorratsbehälter (20) und mit einer die Feststoffe selbstätig in den Gärbehälter (12) einbringenden Beschickungsvorrichtung (18), insbesondere nach einem der vorangehenden Ansprüche,
**dadurch gekennzeichnet,**
**dass** die Beschickungsvorrichtung (18) eine Preßvorrichtung und ein Preßrohr (22) umfaßt, wobei dass Preßrohr (22) in den Gärbehälter (12) mündet.

5. Anlage nach Anspruch 4,
**dadurch gekennzeichnet,**
**dass** das Preßrohr (22) unterhalb einer Schwimmdecke (14) des Gärmediums (10) in den Gärbehälter (12) mündet.

6. Anlage nach einem der Ansprüche 4 oder 5,
**dadurch gekennzeichnet,**
**dass** die Preßvorrichtung ein Aufnahmerohr (24) und einen Preßkolben (28) aufweist, wobei das Aufnahmerohr (24) in das Preßrohr (22) übergeht, und wobei der Durchmesser des Aufnahmerohres (24) größer als der Durchmesser des Preßrohres (22) ausgebildet ist.

7. Anlage nach einem der vorangehenden Ansprüche,
**dadurch gekennzeichnet,**
das am Preßrohr (22) der Beschickungsvorrichtung (18) ein Heizmantel (44) zur Erwärmung der organischen Feststoffe angebracht ist.

8. Anlage nach einem der vorangehenden Ansprüche,
**dadurch gekennzeichnet,**
**dass** am Preßrohr (60) wenigstens eine Sprühvorrichtung (66) zum Einbringen einer Flüssigkeit in das Preßrohr (60) vorgesehen ist.

9. Anlage nach Anspruch 8,
**dadurch gekennzeichnet,**
**dass** die Sprühvorrichtung (66) genau im Knick einer Krümmung (62, 64) des Preßrohres (60) angeordnet ist.

10. Anlage nach Anspruch 8 oder 9,
**dadurch gekennzeichnet,**
**dass** die Sprühvorrichtung (66) im Bereich des Aussenradius der Krümmung (62, 64) des Preßrohres (60) angeordnet ist.

11. Anlage nach einem der Ansprüche 8 bis 10,
**dadurch gekennzeichnet,**
**dass** über den Umfang des Preßrohres (60) verteilt mehrere Sprühvorrichtungen (66) angeordnet sind.

12. Anlage nach einem der Ansprüche 8 bis 11,
**dadurch gekennzeichnet,**
**dass** im Bereich der Krümmung (62) des Preßrohres (60) in Flußrichtung verteilt mehrere Sprühvorrichtungen (66) hintereinander angeordnet sind.

13. Verfahren zur Erzeugung von Biogas mit einer Vorrichtung gemäß einem der vorangehenden Ansprüche,
**dadurch gekennzeichnet,**
**dass** die organischen Feststoffe vor Einbringen in den Gärbehälter (12) auf 50° C bis 90° C, vorzugsweise 70° C erwärmt werden.

14. Verfahren zur Erzeugung von Biogas, insbesondere mit einer Vorrichtung gemäß einem der Ansprüche 4 bis 12,
**dadurch gekennzeichnet,**
**dass** durch Verdichtung der Feststoffe im Pressrohr (22) und/oder im Aufnahmerohr (24) ein gas- und/oder flüssigkeitsdichter Abschluß entsteht.

15. Verfahren zur Erzeugung von Biogas, insbesondere mit einer Vorrichtung gemäß einem der Ansprüche 1 bis 12,
**dadurch gekennzeichnet,**
**dass** die Beschickungsvorrichtung im Chargenbetrieb arbeitet.
